# EUROPEAN PATENT SPECIFICATION

(11) **EP 3 381 388 B1**
(45) Date of publication and mention of the grant of the patent: **23.11.2022**
(21) Application number: 18164633.2
(22) Date of filing: 28.03.2018
(51) Int. Cl.: A61B 17/68, A61B 17/88

(54) **CRANIAL BONE FIXING DEVICE**
KRANIALE KNOCHENFIXIERVORRICHTUNG
DISPOSITIF DE FIXATION D'OS CRÂNIEN

(30) Priority: 31.03.2017 TW 106111257
(43) Date of publication of application: 03.10.2018
(73) Proprietor: OSSAWARE Biotech Co., Ltd., Changhua County 509 (TW)
(72) Inventor: HUANG, Max, Shengang Township, Changhua County 509 (TW)
(74) Representative: Viering, Jentschura & Partner mbB Patent- und Rechtsanwälte

(56) References cited:
- WO-A1-2009/044421
- WO-A2-02/22001
- US-A1- 2002 004 661
- US-A1- 2014 135 852

## Description

### FIELD OF THE INVENTION

The present invention relates to a cranial bone fixing device and a surgical tool thereof, and more particularly to a cranial bone fixing device and a surgical tool thereof that allows for easy, effective, and efficient implantation and removal of the fixing device in a clinic operation.

### BACKGROUND OF THE INVENTION

Fixing devices for cranial bones are known. For example, Taiwan Patent Publication No. 1321463 discloses a fixing device for cranial bones. The fixing device comprises a sleeve, a bolt and a nut. The sleeve has a tube. One end of the tube is provided with a flange, and the other end of the tube is provided with a bottom plate. A through hole is formed in the bottom plate. The bolt has a head and a body extending from the head and inserted through the through hole of the sleeve. The nut is screwed to the body of the bolt and located in the tube of the sleeve.

The aforesaid fixing device is capable of positioning a cranial bone and protecting a patient's head and can be used repeatedly. However, the sleeve, the bolt, and the nut are separate parts and they must be held in sequence with a clamp device before they are implanted and properly positioned. Particularly, after the bolt is implanted, the clamp device must be first released to allow the cranial bone to be returned to the original location, and then the clamp device is operated again to implant the sleeve and the nut. This causes a certain inconvenience in a clinic operation. In addition, after all these parts have been placed in position, it is required to use an additional rotating tool to engage a top slot formed in the nut for rotating the nut to screw and move down along the bolt and thus induce a clamping force between the sleeve and the bolt so as to fix the cranial bone to the cranium securely. This makes it necessary for a clamp tool in implanting and positioning all the parts and another tool used for rotating and tightening. Both implantation and removal operations require the two tools, and the cost is high, and clinic operations become complicated and inconvenient.

In addition, the fixing device is structured by providing a plurality of slots in the top of the nut for engagement with corresponding projections of a surgical tool in order to rotate and drive the nut to screw to the bolt and to drive the sleeve to move down for fixing. However, the surgical tool uses its projections at the lower end thereof to engage the slot in the top end of the slot. The engagement is generally not very secure and would readily lead to undesired separation between the surgical tool and the nut during screwing of the nut, making it necessary for re-positioning for engagement. The operation is very inconvenient.

Further, liquid accumulation often occurs inside the patient's head after the operation. The tissue fluid must be effectively and quickly drained in order to prevent excessive brain pressure and swelling. However, the aforesaid fixing device is generally not equipped with a drainage structure. Thus, surgeons must drill a hole at a neighboring site for insertion and installation of a drain tube. This increases the inconvenience of a surgery operation.

Accordingly, the inventor of the present invention has devoted himself based on his many years of practical experiences to solve these problems. WO 02/22001 A2 dislcoses a cranial bone fixing device, comprising a lower fastening member and a movable retaining assembly; the lower fastening member having a first base part, a threaded rod having an external thread; the movable retaining assembly comprising an upper fastening member and a nut, the upper fastening member has a second base part and a sleeve part, the sleeve part having a pivot portion inside the sleeve part, the nut being received in the sleeve part, the nut having an internal thread to engage with the external thread of the threaded rod, an upper end of the nut having a first positioning portion, an outer surface of the nut being provided with a second pivot portion (41) pivotally connected to the first pivot portion of the upper fastening member such that the nut and the upper fastening member collectively form the movable retaining assembly. Another device is disclosed in US 2002/004661 A1.

### SUMMARY OF THE INVENTION

The primary object of the present invention is to provide a cranial bone fixing device and a surgical tool that effectively simplify the component assembly operation to achieve convenience of easy and efficient completion of the component assembly operation.

According to the present invention, a cranial bone fixing device as defined by claim 1 is provided. The dependent claims show some examples of such a device.

A surgical tool is also described herein but does not form part of the claimed invention. The surgical tool comprises an inner guide rod and an outer sleeve. A lower end of the inner guide rod has a second coupling portion. An upper end of the inner guide rod has a grip portion and a third coupling portion below the grip portion. The outer sleeve has a guide hole for insertion of the inner guide rod. An upper end of an interior of the guide hole is provided with a fourth coupling portion to engage with the third coupling portion of the inner guide rod. When the outer sleeve and the inner guide rod are coupled and positioned through the third coupling portion and the fourth coupling portion, a spacing distance is defined between the outer sleeve and the second coupling portion at the lower end of the inner guide rod. A lower end of the outer sleeve has a second positioning portion.

Preferably, the second coupling portion and the third coupling portion are external threads in opposite directions. The second coupling portion is a right-handed external thread. The third coupling portion is a left-handed external thread. The fourth coupling portion is a left-handed internal thread to engage with the left-handed external thread of the third coupling portion.

Preferably, the third coupling portion of the inner guide rod is a coupling portion that is in a non-circular shape, the fourth coupling portion of the outer sleeve is a positioning slot formed in a top end of the outer sleeve and corresponding in shape to the third coupling portion. The third coupling portion and the fourth coupling portion have an identical non-circular shape, so that the third coupling portion and the fourth coupling portion can engage with each other in a manner of being movable in unison with each other.

Thereby, the upper fastening member and the nut of the present invention are pivotally combined together as a single movable retaining assembly to facilitate engagement and positioning of the entire combination of the movable retaining assembly and the outer sleeve. The outer sleeve is combinable with and positioned on an upper end of the inner guide rod, such that the movable retaining assembly is set to be spaced from an outer surface of a cranium by a spacing distance to facilitate implantation and restoration of the cranial bone. The implantation tool only needs one single surgical tool. The upper and lower ends of the inner guide rod are respectively provided with threads of opposite directions so that by holding and rotating one single component, the outer sleeve, in one direction to move vertically, implantation and removal can be achieved thereby providing the advantages of an efficient and easy way of operation and improving the drawbacks of the prior art.

Embodiments of the present invention will now be described, by way of example only, with reference to the accompanying drawings.

### BRIEF DESCRIPTION OF THE DRAWINGS

FIG. 1 is an exploded view of the present invention;
FIG. 2 is a cross-sectional view of a movable retaining assembly of the present invention;
FIG. 3 is a schematic view illustrating a combination of a cranial bone fixing device according to the invention and a surgical tool;
FIG. 4 is a perspective view showing the cranial bone fixing device of the invention and the surgical tool combined together;
FIG. 5 is a schematic view illustrating an operation of implanting the combination of FIG. 4 in a drilled hole of a cranium;
FIG. 6 is a schematic view illustrating implantation and restoration of a cranial bone of FIG. 5 through rotation and downward movement of an outer sleeve;
FIG. 7 is a schematic view illustrating that the outer sleeve of FIG. 6 is further continuously rotated to drive a nut and an upper fastening member to move downward to achieve securely clamping and fixing;
FIG. 8 is a schematic view illustrating that the outer sleeve of FIG. 7 is moved upward to engage with an inner guide rod and further rotated to have the inner guide rod removed out of a threaded rod;
FIG. 9 is a perspective schematic view illustrating that the surgical tool of FIG. 8 is completely removed;
FIG. 10 is a schematic view illustrating another embodiment of third and fourth coupling portions;
FIG. 11 is a schematic view illustrating a further embodiment of third and fourth coupling portions;
FIG. 12 is a schematic view illustrating the present invention in combination with a drain tube;
FIG. 13 is a schematic view illustrating another embodiment of a combination of an upper fastening member and a lower fastening member according to the present invention;
FIG. 14 is a schematic view illustrating the present invention as shown in FIG. 13 combined with a drain tube;
FIG. 15 is a schematic view illustrating a drain tube inserted through an inner guide rod;
FIG. 16 is a perspective view showing a drain tube being received through and constrained in position by open slits; and
FIG. 17 is a schematic plan view of FIG. 16.

### DETAILED DESCRIPTION OF THE PREFERRED EMBODIMENTS

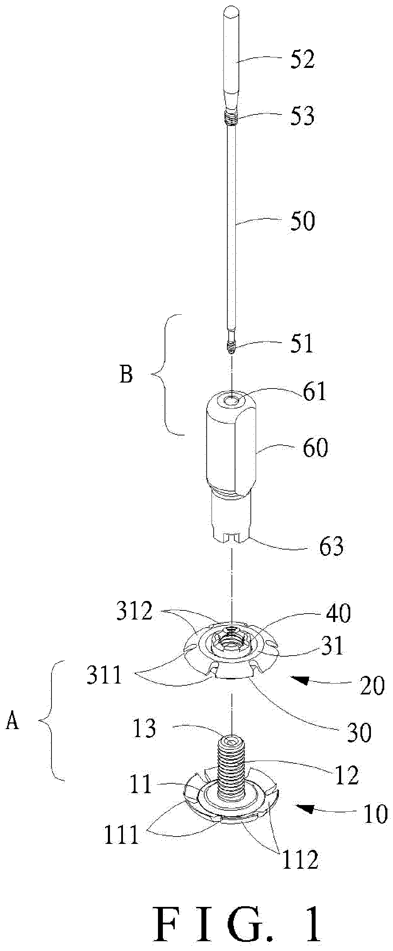

As shown in FIGS. 1-4, the present invention discloses a cranial bone fixing device A and a surgical tool B thereof.

The cranial bone fixing device A comprises a lower fastening member 10 and a movable retaining assembly 20.

The lower fastening member 10 has a first base part 11, a threaded rod 12 having an external thread, and a first coupling portion 13 disposed inside the threaded rod 12. The first base part 11 is in the form of a plate or a disk having an outer circumference that is formed with a plurality of open slits 111 at intervals so as to form a plurality of first petal-like segments 112. The plurality of first petal-like segments 112 have a thickness that is gradually reduced toward its outer circumferential edge for easy insertion into a cranium 70 and a cranial bone 72 for fixing them together. The first coupling portion 13 is an internal thread.

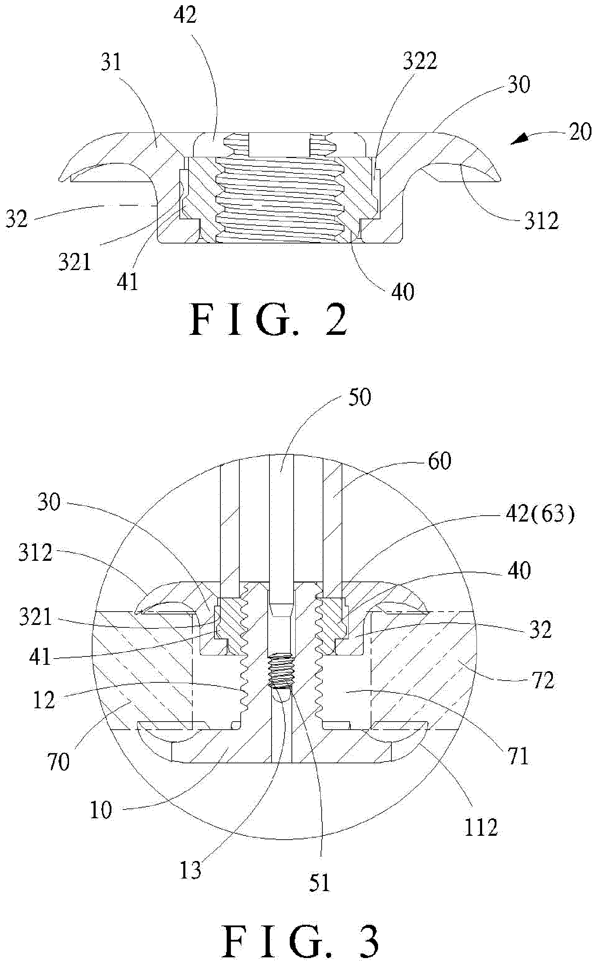

The movable retaining assembly 20 comprises an upper fastening member 30 and a nut 40 threadedly engaged with the external thread of the threaded rod 12. The upper fastening member 30 has a second base part 31 and a sleeve part 32. The sleeve part 32 has a pivot portion 321 inside the sleeve part 32. The second base part 31 is in the form of a plate or a disk having an outer circumference that is formed with a plurality of open slits 311 at intervals so as to form a plurality of second petal-like segments 312. The plurality of second petal-like segments 312 have a thickness that is gradually reduced toward its outer circumferential edge for easy insertion into the cranium 70 and the cranial bone 72 for fixing them together. The sleeve part 32 is an annular sleeve. The sleeve part 32 has a cavity portion 322 having a bottom in which a through hole is formed. The first pivot portion 321 is a recess which is formed by recessing an inner surface of the cavity portion 322. The nut 40 is received in the cavity portion 322 of the sleeve part 32. The nut 40 has an internal thread to engage with the external thread of the threaded rod 12. An upper end of the nut 40 has a first positioning portion 42. The first positioning portion 42 is configured to cooperate with a surgical tool B. Preferably, the first positioning portion 42 is a concave-convex portion circumferentially formed on the upper end of the nut 42 or includes at least two pits (not shown) formed by recessing a lower end surface of the nut 42. An outer surface of the nut 40 is provided with a second pivot portion 41 that is pivotally connected to the first pivot portion 321 of the upper fastening member 3C According to the invention, the second pivot portion 41 is an annular projection received in the recess of the first pivot portion 321, so that the nut 40 and the upper fastening member 30 collectively form the movable retaining assembly, as shown in FIG. 2.

The surgical tool B comprises an inner guide rod 50 and an outer sleeve 60.

A lower end of the inner guide rod 50 has a second coupling portion 51 to engage with the first coupling portion 13 inside the threaded rod 12 of the cranial bone fixing device A. The second coupling portion 51 is an external thread to engage with the internal thread of the first coupling portion 13; or alternatively, the second coupling portion 51 may be a positioning block projecting from the lower end of the inner guide rod 50, and the first coupling portion 13 may be a coupling hole having an L-shaped positioning groove provided on the threaded rod 12 for the positioning block to be inserted, rotated and positioned therein (not illustrated). An upper end of the inner guide rod 50 has a grip portion 52 and a third coupling portion 53 below the grip portion 52. Preferably, the third coupling portion 53 is an external thread.

The outer sleeve 60 has a guide hole 61 for insertion of the inner guide rod 50. An upper end of the interior of the guide hole 61 is provided with a fourth coupling portion 62 to engage with the third coupling portion 53 of the inner guide rod 50. When the outer sleeve 60 is coupled to and positioned with respect to the inner guide rod 50, a spacing distance X is defined between the outer sleeve 60 and the second coupling portion 51 of the inner guide rod 50. The fourth coupling portion 62 is an internal thread to engage with the external thread of the third coupling portion 53. The thread direction of the second coupling portion 51 is opposite to the thread direction of the third coupling portion 53 and the fourth coupling portion 62. Preferably, the second coupling portion 51 is a right-handed thread, and the third coupling portion 53 and the fourth coupling portion 62 are left-handed threads. A lower end of the outer sleeve 60 has a second positioning portion 63. The second positioning portion 63 is concave-convex portion circumferentially formed on the lower end of the outer sleeve 60 to correspond to and mate with the concave-convex portion of the first positioning portion 42 so as to be connected with and drive the nut 40 to move in unison therewith; or alternatively, a lower end surface of the outer sleeve 60 is provided with two posts corresponding to and mating with the two pits of the first positioning portion 42 for driving the nut 40 to move in unison therewith (not illustrated).

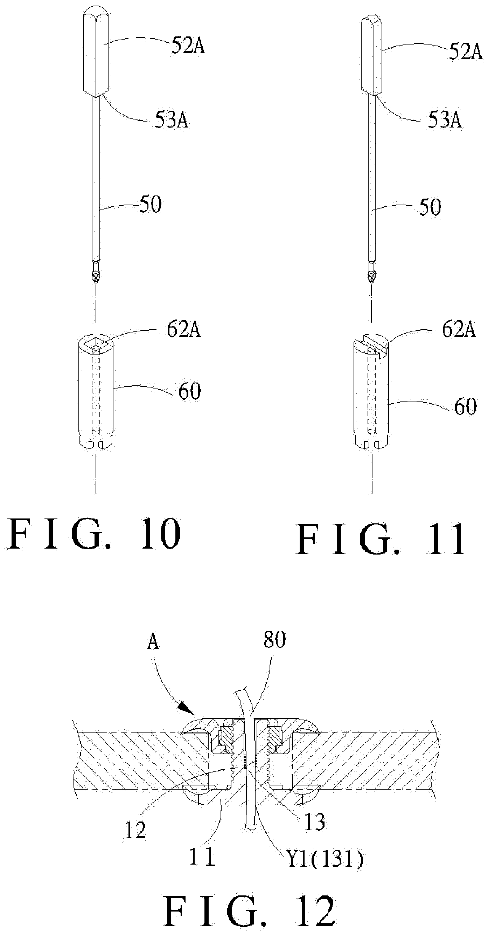

FIGS. 10 and 11 are schematic views of another example of the third coupling portion and the fourth coupling portion. In this example, the third coupling portion 53A of the inner guide rod 50 may be a coupling portion that is in a non-circular shape, and the fourth coupling portion 62A of the outer sleeve 60 is a positioning slot formed in a top end of the outer sleeve 60 and corresponding in shape to the third coupling portion 53A, such as a linear slot or a rectangular slot as shown in the drawings. In other words, the third coupling portion 53A and the fourth coupling portion 62A have an identical non-circular shape, so that the third coupling portion 53A and the fourth coupling portion 62A can mate and couple with each other in a manner of being movable in unison with each other. Further, the grip portion 52A may have the same shape as that of the third coupling portion so that the third coupling portion 53A may be formed by directly extending the grip portion 52A.

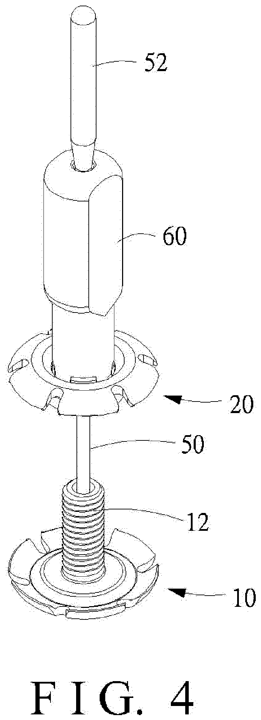

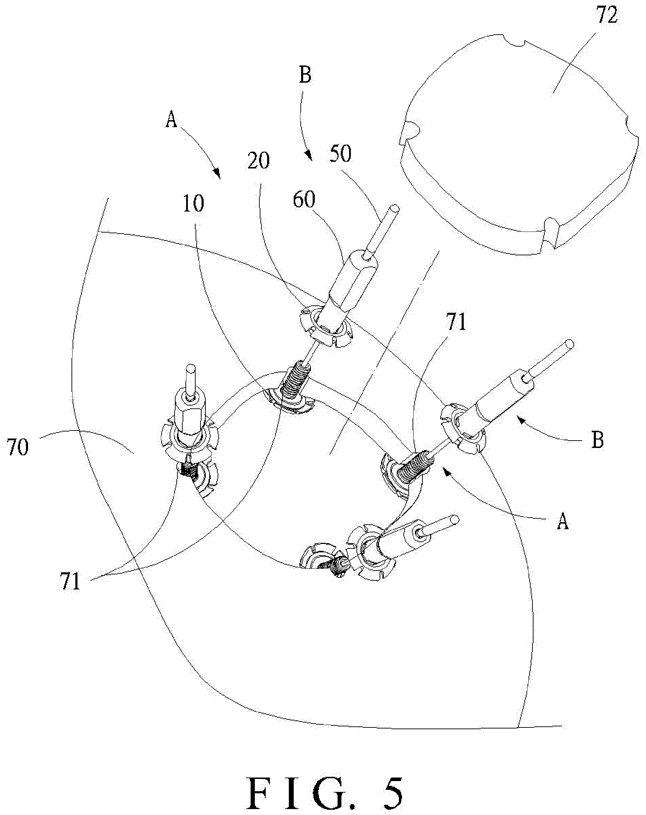

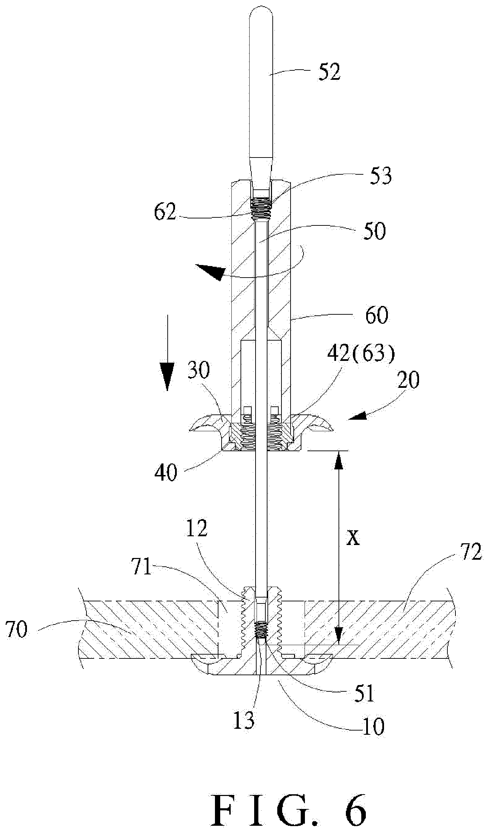

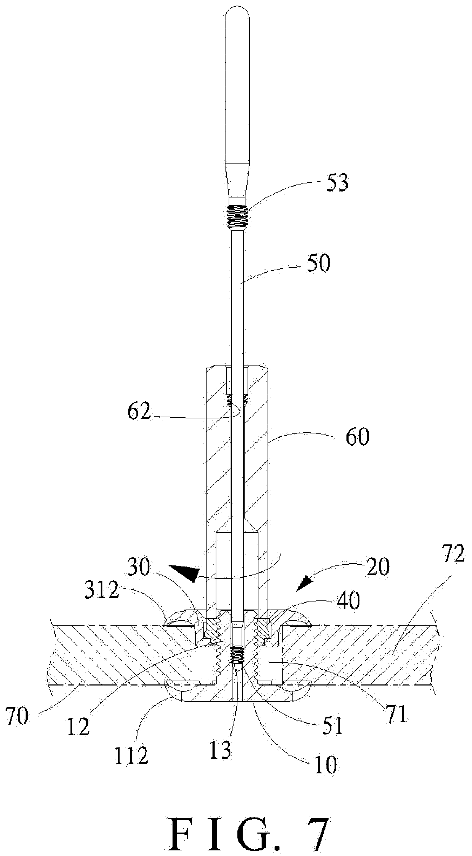

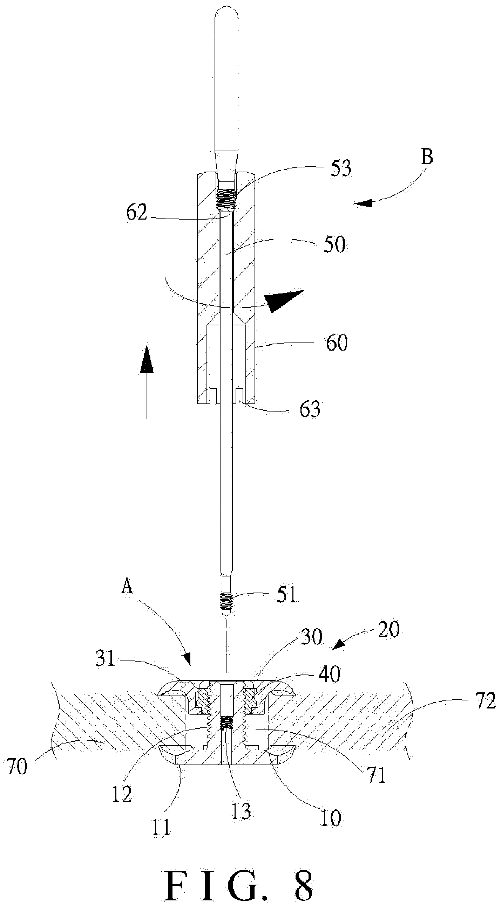

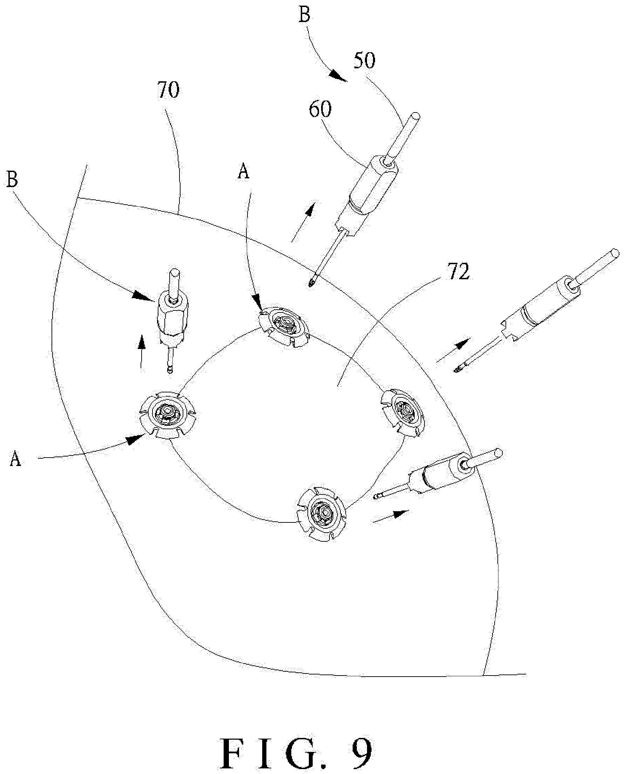

FIGS. 4-9 are schematic views, illustrating the assembly of the cranial bone fixing device A and the surgical tool B and an implantation operation conducted therewith. In this example, as shown in FIG. 4, it only needs to insert the inner guide rod 50 through the outer sleeve 60 and the movable retaining assembly 20 to have the second coupling portion 51 provided on the lower end thereof screwed to the first coupling portion 13 inside the threaded rod 12 and the fourth coupling portion 62 provided on the upper end of the outer sleeve 60 screwed to the third coupling portion 53 provided on the upper end of the inner guide rod 50 to achieve positioning. The movable retaining assembly 20 is coupled through the engagement between the first positioning portion 42 provided on the upper end of the nut 40 and the second positioning portion 63 provided on the lower end of the outer sleeve 60 such that the cranial bone fixing device A and the surgical tool B are combined with and positioned with respect to each other to form a combined assembly. As shown in FIG. 5, the cranial bone fixing device A and the surgical tool B can be placed in a drilled hole 71 of the cranium 70 in an easy and convenient way. Further, as shown in FIG. 6, the outer sleeve 60 and the second coupling portion 51 of the inner guide rod 50 have the spacing distance X defined therebetween such that when the outer sleeve 60 is combined with the movable retaining assembly 20, the movable retaining assembly 20 is arranged in a manner of being spaced from an outer surface of the cranium 70 by a distance to facilitate the resetting of the cranial bone 72. As shown in FIGS. 6 and 7, after the cranial bone 72 is set for closure, the outer sleeve 60 is rotated and thus moved downward, where driving engagement between the second positioning portion 63 provided on the lower end thereof and the first positioning portion 42 of the nut 40 allows the outer sleeve 60 that is being rotated to drive the nut 40 and the threaded rod 12 to screw with each other and move downward thereby driving and tightening the upper fastening member 30 and the lower fastening member 10 to generate a clamping force for effectively and securely clamping and fixing the cranial bone 72 in position on the cranium 70. As shown in FIG. 8, the outer sleeve 60 may then be moved upward through a reverse rotation so that the fourth coupling portion 62 and the third coupling portion 53 of the inner guide rod 50 engage with each other, and through a further and continuous rotation, the second coupling portion 51 provided on the lower end of the inner guide rod 50 is screwed out of and thus detached from the inner threaded hole of the first coupling portion 13 of the threaded rod 12, allowing the surgical tool B to be quickly removed from the cranial bone fixing device A, as shown in FIG. 9. On the other hand, to remove the cranial bone fixing device A or the cranial bone 72, it only needs to conduct the operation in an opposite way to remove the cranial bone fixing device A from the cranium 70. The present surgical tool is easy and convenient in operation for both implantation and removal.

The cranial bone fixing device A according to the invention and the surgical tool B, when compared with the prior art, provide the following advantages:
(1) The nut 40 and the upper fastening member 30 of the present invention are combined with each other in a rotatable manner to form the movable retaining assembly 20. This simplifies an assembly process of parts. Further, the cranial bone fixing device A and the surgical tool B are combined, in advance, as a combined assembly so that, without the use of any tool, the entire combined assembly of the cranial bone fixing device A and the surgical tool B can be implanted together so as to make an implantation operation easy and efficient. The drawback of the prior art of being inconvenient in use, where clamping tools are necessary for individually picking, clamping and implanting parts of a fixing device one by one.
(2) When the outer sleeve 60 is screwed to and positioned with respect to the upper end of the inner guide rod 50, the outer sleeve 60 and the second coupling portion 51 of the inner guide rod 50 have the spacing distance X defined therebetween. The nut 40 and the upper fastening member 30 are rotatably combined to form the movable retaining assembly 20, so that the movable retaining assembly 20, in the entirety thereof, can be coupled to and positioned with respect to the outer sleeve 60 such that the spacing distance X provides a sufficient space to allow the movable retaining assembly 20 and the outer surface of the cranium 70 to space from each other by a distance for easy insertion and restoration of the cranial bone 72.
(3) The second positioning portion 51 and third positioning portion 53 provided on the lower end and the upper end of the inner guide rod 50 are provided with threads in opposite directions, so that rotating and displacing the outer sleeve 60 with a single hand enables the upper fastening member 30 and the lower fastening member 10 to press against the cranial bone 72 and the cranium 70 for securely clamping and positioning. Further, to remove the surgical tool B, it only needs to move the outer sleeve 60 upward through a reverse rotation to be thus removed from the cranial bone fixing device A. The present surgical tool allows for holding, with one single hand, one single component, the outer sleeve 60, to conduct an operation to achieve ease and convenience of fast and smooth fastening and tightening the cranial bone fixing device A and quick removal of the surgical tool B. Further, to remove the cranial bone fixing device A, it only needs to operate in an opposite way. Thus, both implantation and removal can be achieved by using one surgical tool with one hand to hold one single component, so that clinic operations are efficient and easy. The drawbacks of the prior art of being complicated and inconvenient in operation where two different surgical tools must be used respectively for clamping and fastening.
(4) In the present surgical tool, in addition to the second positioning portion 63 provided on the lower end of the outer sleeve 60 to mate with the first positioning portion 42 of the nut 40, the outer sleeve 60 is provided with the guide hole 61 for insertion of the inner guide rod 50 so as to have additional support with the inner guide rod 50 to enhance the rotational stability of the outer sleeve 60 without any concern of undesired detachment, and the inconvenience of reengaging the outer sleeve 60 with the nut 40 due to undesired detachment can be prevented.
(5) The present invention uses the upper fastening member to cover the drilled hole 71, and tightening can be achieved by rotating the nut 40 for screwing with the threaded rod 12. This way can prevent the outside surfaces of the cranium 70 and the cranial bone 72 from being damaged due to rotation of the upper fastening member 30, thereby achieving positioning of the cranial bone without hurting the patient's head and allowing for repeated use.

Further referring to FIG. 12, the cranial bone fixing device A of the present invention is further provided with a drain channel Y1. The drain channel Y1 is formed by extending from the internal thread of the first coupling portion 13 to penetrate through the first base part 11 to form a through hole 131. The through hole 131 allows a drain tube 80 to insert therethrough for draining tissue fluid inside the brain, thereby preventing excessive pressure and swelling of the brain due to accumulation of fluid.

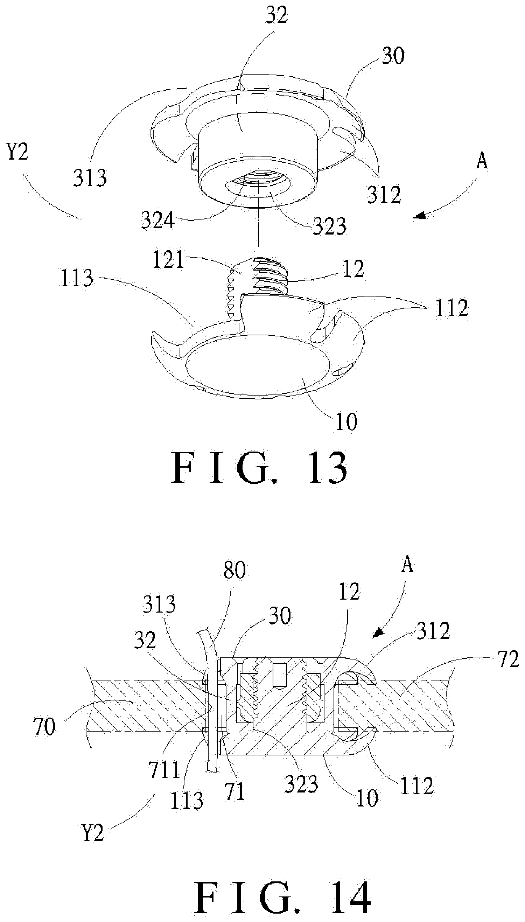

As shown in FIGS. 13 and 14, another embodiment that the cranial bone fixing device A comprises a drain channel is illustrated. The cranial bone fixing device A can be mounted in the drilled hole 71 of the cranium 70 and the drain channel Y2 is formed in such a way that one of the plurality of first petal-like segments 312 of the upper fastening member 30 and a corresponding one of the plurality of second petal-like segments 112 of the lower fastening member 10 are cut to form a first notch 313 and a second notch 113, respectively. The sleeve part 32 of the upper fastening member 30 is formed with a perforation 323 having an internal cut surface 324. The threaded rod 12 of the lower fastening member 10 is provided with an external cut surface 121 corresponding to the internal cut surface 324. When the threaded rod 12 is fitted into the perforation 323, the first notch 313 is aligned with the second notch 113. Further, the drilled hole 71 of the cranium 70 is provided with an aperture 711 extending therethrough and corresponding to and in communication with the first notch 313 and the second notch 113 to receive a drain tube 80 to extend, vertically, through the first notch 313, the aperture 711, and the second notch 113 to drain brain tissue fluid, thereby preventing excessive pressure and swelling of the brain due to accumulation of fluid.

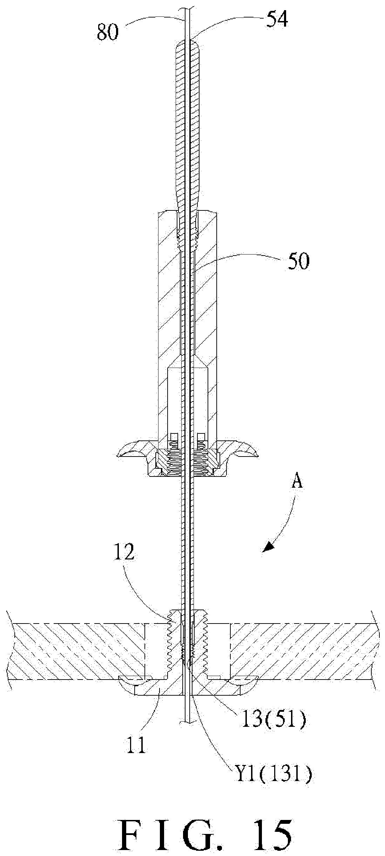

As shown in FIG. 15, a through passage 54 is defined in the inner guide rod 50. The cranial bone fixing device A is provided with a drain channel Y1, similar to the aforesaid. The drain channel Y1 is formed by extending from the internal thread of the first coupling portion 13 to penetrate through the first base part 11 to form a through hole 131. When the external thread of the second coupling portion 51 of the inner guide rod 50 is engaged with the internal thread of the first coupling portion 13 of the threaded rod 12, the through passage 54 and the drain channel Y1 correspond to and communicate with each other to receive a drain tube 80 for draining brain tissue fluid to prevent excessive pressure and swelling of the brain due to accumulation of fluid.

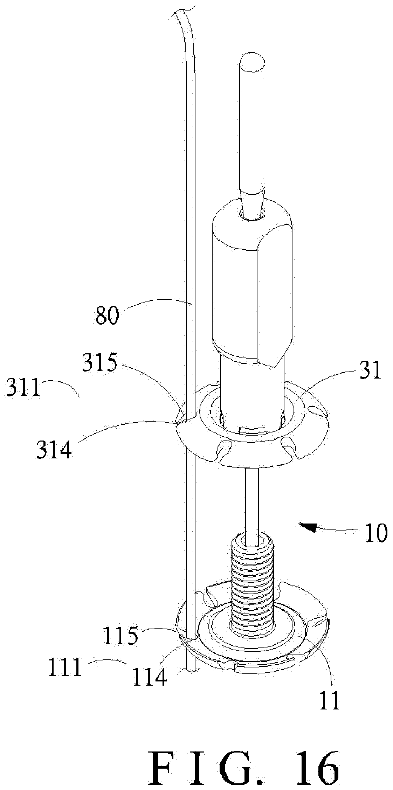

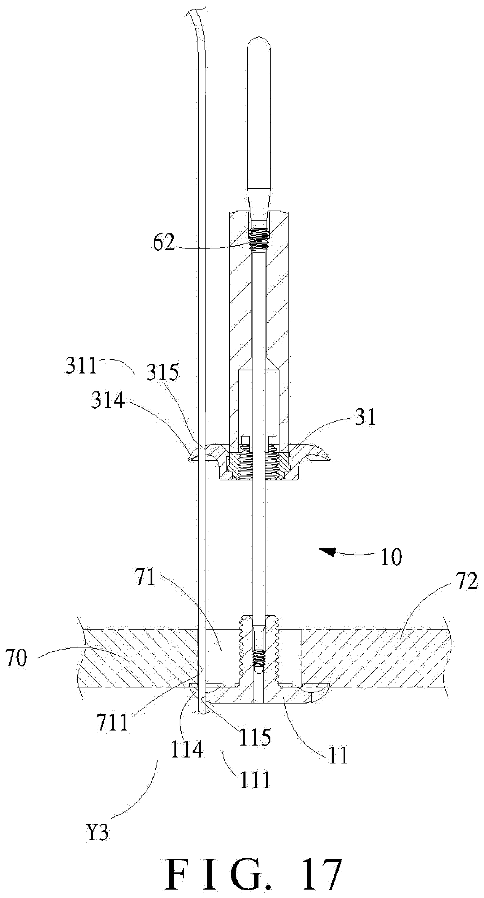

As shown in FIGS. 16 and 17, the cranial bone fixing device A is mounted at the drilled hole 71 of the cranium 70. The cranial bone fixing device A comprises a drain channel Y3. The drain channel Y3 is formed of the open slits 111, 311 of the first base part 11 and the second base part 31 in alignment with each other in a vertical direction for insertion of a drain tube 80. An aperture 711 is formed in the drilled hole 71 of the cranium 70 and corresponding to the open slits 111, 311 so as to allow the drain tube 80 arranged between the two open slits 111, 311 to be received in the aperture 711, whereby brain tissue fluid can be drained through the drain tube 80 to prevent excessive pressure and swelling of the brain due to accumulation of fluid. Further, the open slits 111, 311 are in the form of a keyway, having an opening 114, 314 less than the tube diameter of the drain tube 80 and a limit hole 115, 315 greater than the tube diameter of the drain tube 80 to allow the drain tube 80 to be received and positioned therein.

In summary, the cranial bone fixing device according to the invention and the surgical tool combine the upper fastening member with the nut to form the movable retaining assembly. The implantation tool only needs one single surgical tool. The upper and lower ends of the inner guide rod are respectively provided with threads in opposite directions, so that by holding and rotating one single component, the outer sleeve, in one direction to move up and down, implantation and removal can be achieved, thereby providing the advantages of an efficient and easy way of operation.

Although particular embodiments of the present invention have been described in detail for purposes of illustration, various modifications and enhancements may be made without departing from the scope of the present invention. Accordingly, the present invention is not to be limited except as by the appended claims.

## Claims

1. A cranial bone fixing device (A), comprising a lower fastening member (10) and a movable retaining assembly (20); the lower fastening member (10) having a first base part (11), a threaded rod (12) having an external thread; the movable retaining assembly (20) comprising an upper fastening member (30) and a nut (40), the upper fastening member (30) has a second base part (31) and a sleeve part (32), the sleeve part (32) being an annular sleeve and having a cavity portion (322), the sleeve part (32) having a pivot portion (321) inside the sleeve part (32), the nut (40) being received in the sleeve part (32), the nut (40) having an internal thread to engage with the external thread of the threaded rod (12), an upper end of the nut (40) having a first positioning portion (42), an outer surface of the nut (40) being provided with a second pivot portion (41) pivotally connected to the first pivot portion (321) of the upper fastening member (30) such that the nut (40) and the upper fastening member (30) collectively form the movable retaining assembly (20)
**characterized in that**
the threaded rod (12) of the lower fastening member (10) has a first coupling portion (13) disposed inside the threaded rod (12),
and **in that** the cavity portion (322) of the sleeve part (32) has a bottom in which a through hole is formed, the first pivot portion (321) being a recess which is formed by recessing an inner surface of the cavity portion (322), and the second pivot portion (41) being an annular projection engaged in the recess of the first pivot portion (321), thereby pivotally combining together the upper fastening member (30) and the nut (40) as a single movable retaining assembly.

2. The cranial bone fixing device (A) as claimed in claim 1, further having a drain channel (Y1, Y2, Y3) for insertion of a drain tube (80).

3. The cranial bone fixing device (A) as claimed in claim 1, wherein the first coupling portion (13) is an internal thread.

4. The cranial bone fixing device (A) as claimed in claim 1, wherein the first positioning portion (42) is a concave-convex portion circumferentially formed on the upper end of the nut (42) or includes at least two pits formed by recessing an end surface of the nut (42).

5. The cranial bone fixing device (A) as claimed in claim 2, wherein the first base part (11) and the second base part (31) are in the form of a plate or a disk having an outer circumference that is formed with a plurality of open slits (111, 311) at intervals so as to form a plurality of first petal-like segments (112) and a plurality of second petal-like segments (312) respectively, and the plurality of first petal-like segments (112) and the plurality of second petal-like segments (312) have a thickness that is gradually reduced toward its outer circumferential edge.

6. The cranial bone fixing device (A) as claimed in claim 2, wherein the drain channel (Y1) is formed by extending from the internal thread of the first coupling portion (13) to penetrate through the first base part (11) to form a through hole (131).

7. The cranial bone fixing device (A) as claimed in claim 5, wherein the cranial bone fixing device (A) is mountable in a drilled hole (71) of a cranium (70), the drain channel (Y2) is formed of a first notch (313) and a second notch (113) formed by cutting one of the plurality of first petal-like segments (312) of the upper fastening member (30) and a corresponding one of the plurality of second petal-like segments (112) of the lower fastening member (10), the sleeve part (32) of the upper fastening member (30) is formed with a perforation (323) having an internal cut surface (324), the threaded rod (12) of the lower fastening member (10) is provided with an external cut surface (121) corresponding to the internal cut surface (324), when the threaded rod (12) is fitted into the perforation (323), the first notch (313) is aligned with the second notch (113), the drilled hole (71) of the cranium (70) is provided with an aperture (711) extending therethrough and corresponding to and in communication with the first notch (313) and the second notch (113) for insertion of the drain tube (80) to drain brain tissue fluid, thereby preventing excessive pressure and swelling of the brain due to accumulation of fluid.

8. The cranial bone fixing device (A) as claimed in claim 5, wherein the cranial bone fixing device (A) is mountable in a drilled hole (71) of a cranium (70), the drain channel (Y3) is formed of the open slits (111, 311) of the first base part (11) and the second base part (31) in alignment with each other in a vertical direction for insertion of the drain tube (80), an aperture (711) is formed in the drilled hole (71) of the cranium (70) and corresponding to the open slits (111, 311) so as to allow the drain tube (80) arranged between the two open slits (111, 311) to be received in the aperture (711).

9. The cranial bone fixing device (A) as claimed in claim 8, wherein the open slits (111, 311) are in the form of a keyway, having an opening (114, 314) less than a tube diameter of the drain tube (80) and a limit hole (115, 315) greater than the tube diameter of the drain tube (80).

## Patentansprüche

1. Eine Fixiervorrichtung für Schädelknochen (A), umfassend ein unteres Befestigungselement (10) und eine bewegliche Haltevorrichtung (20); das untere Befestigungselement (10) aus einem ersten Basisteil (11) besteht und eine Gewindestange (12) mit einem Außengewinde aufweist; die bewegliche Haltevorrichtung (20) ein oberes Befestigungselement (30) und eine Mutter (40) umfasst, wobei das obere Befestigungselement (30) aus einem zweiten Basisteil (31) und einem Manschettenteil (32) besteht; das Manschettenteil (32) eine ringförmige Manschette ist und einen Hohlraumteil (322) aufweist; das Manschettenteil (32) innerhalb dieses einen Zapfenteil (321) aufweist; die Mutter (40) im Manschettenteil (32) aufgenommen ist, wobei die Mutter (40) ein Innengewinde aufweist, um mit dem Außengewinde der Gewindestange (12) in Eingriff gebracht zu werden; ein oberes Ende der Mutter (40) einen ersten Positionierungsteil (42) aufweist, während eine Außenfläche der Mutter (40) einen zweiten Zapfenteil (41) aufweist, die schwenkbar am ersten Zapfenteil (321) des oberen Befestigungselements (30) befestigt ist, so dass die Mutter (40) und das obere Befestigungselement (30) zusammen die bewegliche Haltvorrichtung (20) bilden,
**dadurch gekennzeichnet, dass**
die Gewindestange (12) des unteren Befestigungselements (10) einen ersten Kupplungsteil (13) aufweist, der in der Gewindestange (12) angeordnet ist,
und dadurch, dass
der Hohlraumteil (322) des Manschettenteils (32) einen Boden aufweist, in dem ein Durchgangsloch gebildet ist; der erste Zapfenteil (321) eine Aussparung ist, die durch Aussparung einer Innenfläche des Hohlraumteils (322) gebildet ist, während der zweite Zapfenteil (41) ein ringförmiger Vorsprung ist, der in die Aussparung des ersten Zapfenteils (321) eingreift, wodurch das obere Befestigungselement (30) und die Mutter (40) als bewegliche Haltevorrichtung aus Schindel schwenkbar aneinander befestigt sind.

2. Die Fixiervorrichtung für Schädelknochen (A) nach Anspruch 1, die weiter eine Ablaufrinne (Y1, Y2, Y3) aufweist, um in dieser einen Ablaufschlauch (80) einzusetzen.

3. Die Fixiervorrichtung für Schädelknochen (A) nach Anspruch 1, wobei der erste Kupplungsteil (13) ein Innengewinde ist.

4. Die Fixiervorrichtung für Schädelknochen (A) nach Anspruch 1, wobei der erste Positionierungsteil (42) ein konkav-konvexer Teil ist, der umgehend am oberen Ende der Mutter (42) gebildet ist, oder mindestens zwei Vertiefungen aufweist, die durch Aussparen einer Endfläche der Mutter (42) gebildet sind.

5. Die Fixiervorrichtung für Schädelknochen (A) nach Anspruch 2, wobei der erste Basisteil (11) und der zweite Basisteil (31) in Form einer Platte oder Scheibe mit einem Außenumfang gebildet sind, wobei der Außenumfang mit mehreren offenen Schlitzöffnungen (111, 113) in Abständen zueinander gebildet sind, um mehrere erste blütenblattähnliche Segmente (112) bzw. mehrere zweite blütenblattähnliche Segmente (312) zu bilden; die mehreren ersten blütenblattähnlichen Segmente (112) und die mehreren zweiten blütenblattähnliche Segmente (312) eine Dicke aufweisen, die zu deren Außenumfangskante allmählich verringert ist.

6. Die Fixiervorrichtung für Schädelknochen (A) nach Anspruch 2, wobei die Ablaufrinne (Y1) dadurch gebildet ist, dass diese sich vom Innengewinde des ersten Kupplungsteils (13) aus erstreckt, um durch den ersten Basisteil (11) zu verlaufen und ein Durchgangsloch (131) zu bilden.

7. Die Fixiervorrichtung für Schädelknochen (A) nach Anspruch 5, wobei die Fixiervorrichtung für Schädelknochen (A) in das gebohrte Loch (71) eines Schädels (70) montierbar ist; die Ablaufrinne (Y2) mit einer ersten Kerbe (313) und einer zweiten Kerbe (113), die durch Schneiden eines der mehreren ersten blütenblattähnlichen Segmente (312) des oberen Befestigungselements (30) und einem entsprechenden aus mehreren zweiten blütenblattähnlichen Segmente (112) des unteren Befestigungselements (10) gebildet sind; der Manschettenteil (32) des oberen Befestigungselements (30) mit einer Lochung (323) mit einer inneren Schnittfläche (324) gebildet ist, die Gewindestange (12) des unteren Befestigungselements (10) eine äußere Schnittfläche (121) aufweist, die der inneren Schnittfläche (324) entspricht, die erste Kerbe (313) mit der zweiten Kerbe (113) ausgerichtet ist, wenn die Gewindestange (12) in die Lochung (323) eingesetzt ist; das gebohrte Loch (71) im Schädel (70) mit einer Öffnung (711) versehen ist, die durch dieses hindurch verläuft und mit der ersten Kerbe (313) und der zweiten Kerbe (113) übereinstimmt und mit der ersten Kerbe (313) verbunden ist, um den Ablaufschlauch (80) einzuführen und dadurch die Flüssigkeit des Hirngewebes abzuleiten, um dadurch einen übermäßigen Druck und ein Anschwellen des Gehirns aufgrund einer Flüssigkeitsansammlung zu vermeiden.

8. Die Fixiervorrichtung für Schädelknochen (A) nach Anspruch 5, wobei die Fixiervorrichtung für Schädelknochen (A) in einem gebohrten Loch (71) im Schädel (70) montierbar ist; die Ablaufrinne (Y3) aus den offenen Schlitzöffnungen (111, 311) des ersten Basisteils (11) und des zweiten Basisteils (31) in vertikaler Richtung fluchtend zueinander gebildet ist, um den Ablaufschlauch (80) einzuführen; im gebohrten Loch (71) im Schädel (70) eine Öffnung (711) gebildet ist, die mit den offenen Schlitzöffnungen (111, 311) übereinstimmt, damit der zwischen den beiden offenen Schlitzöffnungen (111, 311) angeordnete Ablaufschlauch (80) in der Öffnung (711) aufgenommen werden kann.

9. Die Fixiervorrichtung für Schädelknochen (A) nach Anspruch 8, wobei die offenen Schlitzöffnungen (111, 311) die Form einer Keilnut aufweist und weiter eine Öffnung (114, 314), die kleiner als ein Rohrdurchmesser des Ablaufschlauchs (80) ist, und ein Grenzloch (115, 315), das größer als der Rohrdurchmesser des Ablaufschlauchs (80) ist, aufweisen.

## Revendications

1. Dispositif de fixation pour os crânien (A), **caractérisé par le fait qu'**il comprend un élément de fixation inférieur (10) et un ensemble de retenue mobile (20) ; l'élément de fixation inférieur (10) comportant une première partie de base (11), une tige filetée (12) comportant un filetage extérieur ; l'ensemble de retenue mobile (20) **caractérisé par le fait qu'**il comprend un élément de fixation supérieur (30) et un écrou (40), l'élément de fixation supérieur (30) comporte une seconde partie de base (31) et une partie manchon (32), la partie manchon (32) étant un manchon annulaire et comportant une portion de cavité (322), la partie manchon (32) comportant une première partie pivot (321) à l'intérieur de la partie manchon (32), l'écrou (40) étant reçu dans la partie manchon (32), l'écrou (40) comportant un filetage intérieur pour s'engager avec le filetage extérieur de la tige filetée (12), une extrémité supérieure de l'écrou (40) comportant une première partie de positionnement (42), une surface extérieure de l'écrou (40) étant munie d'une seconde partie pivot (41) raccordée de manière pivotante à la première partie pivot (321) de l'élément de fixation supérieur (30) de sorte que l'écrou (40) et l'élément de fixation supérieur (30) forment collectivement l'ensemble de retenue mobile (20)
**caractérisé en ce que**
la tige filetée (12) de l'élément de fixation inférieur (10) comporte une première partie d'accouplement (13) placée à l'intérieur de la tige filetée (12),
et **en ce que**
la portion de cavité (322) de la partie manchon (32) comporte un fond dans lequel un trou traversant est pratiqué, la première partie pivot (321) étant un évidement formé par l'évidement d'une surface intérieure de la portion de cavité (322), et la seconde partie pivot (41) étant une saillie annulaire engagée dans l'évidement de la première partie pivot (321), combinant ainsi de manière pivotante l'élément de fixation supérieur (30) et l'écrou (40) comme un ensemble de retenue mobile unique.

2. Dispositif de fixation pour os crânien (A) selon la revendication 1, **caractérisé par le fait qu'**il comprend en outre un canal de drainage (Y1, Y2, Y3) pour l'insertion d'un tube de drainage (80).

3. Dispositif de fixation pour os crânien (A) selon la revendication 1, **caractérisé par le fait que** la première partie d'accouplement (13) est un filetage intérieur.

4. Dispositif de fixation pour os crânien (A) selon la revendication 1, **caractérisé par le fait que** la première partie de positionnement (42) est une partie concave-convexe formée circonférentiellement sur l'extrémité supérieure de l'écrou (42) ou comprend au moins deux creux formés en évidant une surface d'extrémité de l'écrou (42).

5. Dispositif de fixation pour os crânien (A) selon la revendication 2, **caractérisé par le fait que** la première partie de base (11) et la seconde partie de base (31) se présentent sous la forme d'une plaque ou d'un disque comportant une circonférence extérieure formée d'une pluralité de fentes ouvertes (111, 311) à intervalles de manière à former respectivement une pluralité de premiers segments en forme de pétales (112) et une pluralité de seconds segments en forme de pétales (312), et la pluralité de premiers segments en forme de pétales (112) et la pluralité de seconds segments en forme de pétales (312) présentent une épaisseur qui se réduit progressivement vers leur bord circonférentiel extérieur.

6. Dispositif de fixation pour os crânien (A) selon la revendication 2, **caractérisé par le fait que** le canal de drainage (Y1) est formé en s'étendant du filetage interne de la première partie d'accouplement (13) pour pénétrer à travers la première partie de base (11) pour former un trou traversant (131).

7. Dispositif de fixation pour os crânien (A) selon la revendication 5, **caractérisé par le fait que** dispositif de fixation pour os crânien (A) est montable dans un trou foré (71) d'un crâne (70), le canal de drainage (Y2) est formé d'une première encoche (313) et d'une seconde encoche (113) formées en coupant l'un de la pluralité de premiers segments en forme de pétale (312) de l'élément de fixation supérieur (30) et un segment correspondant de la pluralité de seconds segments en forme de pétale (112) de l'élément de fixation inférieur (10), la partie manchon (32) de l'élément de fixation supérieur (30) comporte une perforation (323) comportant une surface de découpe intérieure (324), la tige filetée (12) de l'élément de fixation inférieur (10) est pourvue d'une surface de découpe extérieure (121) correspondant à la surface de découpe intérieure (324), lorsque la tige filetée (12) est insérée dans la perforation (323), la première encoche (313) est alignée avec la seconde encoche (113), le trou foré (71) du crâne (70) est muni d'une ouverture (711) s'étendant à travers et correspondant à et en communication avec la première encoche (313) et la seconde encoche (113) pour l'insertion du tube de drainage (80) pour drainer le liquide du tissu cérébral, empêchant ainsi une pression excessive et un gonflement du cerveau dû à l'accumulation de liquide.

8. Dispositif de fixation pour os crânien (A) selon la revendication 5, **caractérisé par le fait que** dispositif de fixation pour os crânien (A) est montable dans un trou foré (71) d'un crâne (70), le canal de drainage (Y3) est formé des fentes ouvertes (111, 311) de la première partie de base (11) et de la seconde partie de base (31) en alignement les unes avec les autres dans une direction verticale pour l'insertion du tube de drainage (80), une ouverture (711) est formée dans le trou foré (71) du crâne (70) et correspond aux fentes ouvertes (111, 311) de manière à permettre la réception du tube de drainage (80) placé entre les deux fentes ouvertes (111, 311) dans l'ouverture (711).

9. Dispositif de fixation pour os crânien (A) selon la revendication 8, **caractérisé par le fait que** les fentes ouvertes (111, 311) sont en forme de clavette, comportant une ouverture (114, 314) inférieure au diamètre du tube de drainage (80) et un trou de limitation (115, 315) supérieur au diamètre du tube de drainage (80).
